# EUROPEAN PATENT APPLICATION

(11) **EP 4 026 513 A1**
(43) Date of publication of application: **13.07.2022**
(21) Application number: 20860451.2
(22) Date of filing: 01.09.2020
(51) Int. Cl.: A61C 8/00, A61C 19/04

(54) **TREATMENT SUPPORT SYSTEM AND ALIGNMENT DEVICE**

(30) Priority: 02.09.2019 JP 2019159800
(71) Applicant: Safe Approach Medical Inc., Fukuoka-shi, Fukuoka 814-0001 (JP)
(72) Inventor: CHO, Byunghyun, Fukuoka-shi, Fukuoka 812-0054 (JP)
(74) Representative: Plougmann Vingtoft a/s
(86) International application number: PCT/JP2020/033138
(87) International publication number: WO 2021/045062

(57) **Abstract**

A CT apparatus 120 is a so-called CT scanning apparatus that captures a tomographic image of the whole or a part of the area from the upper jaw to the lower jaw of a patient, and outputs CT data (captured image data). An oral cavity scanner 200 is an apparatus that obtains a stereoscopic image by optically capturing an image of the inside of an oral cavity in a state that a plate 110 is fixed to teeth, and outputs the stereoscopic image as three-dimensional shape data. A processing apparatus 190 computes a positional relationship between the teeth and fiducial frame markers on the basis of the CT data and three-dimensional shape data that are obtained by image-capturing performed in a state that the plate 110 is put on the teeth, and a positional relationship between plate markers and the fiducial frame markers.

## Description

### TECHNICAL FIELD

The present invention relates to a surgical procedure support system and a registration apparatus that support placement of to-be-embedded objects such as implants.

### BACKGROUND OF THE INVENTION

There are known surgical procedure support systems that display the current position of a drill blade over a pre-captured design image at time of placement of a to-be-embedded object such as an implant or a transplant donor target tooth. When the design image is captured, a drill blade for designing is positioned on a plaster model, and a depth and shape of a hole that should be cut for embedment of the implant or transplant donor target tooth are decided (Patent Document 1).

### PRIOR ART

### PATENT DOCUMENTS

Patent Document 1: WO-A-2018/088146

### SUMMARY OF INVENTION

### PROBLEMS TO BE SOLVED BY THE INVENTION

However, if a drill blade which is different from a drill blade to be used for cutting the hole for the implant is used, replacement of the drill blades is necessary at time of the designing and at time of the cutting, and this is cumbersome. In addition, there has been a fear that a practitioner cannot grasp the current angle and position of the drill blade accurately at time of the actual cutting.

The present invention has been made in view of these problems, and an object thereof is to provide a surgical procedure support system and a registration apparatus that support a practitioner such that holes where implants are to be placed can be created accurately.

### MEANS FOR SOLVING THE PROBLEMS

A surgical procedure support system according to a first invention of the present application includes: a plate that is to be put on teeth, and has a plate marker; an image capturing section that captures an image of a state that the plate is put on the teeth, and outputs captured image data; an oral cavity scanner that acquires a three-dimensional shape in an oral cavity in a state that the plate is put on the teeth, and outputs three-dimensional shape data; and a processing apparatus that computes an embedment completion position of a to-be-embedded object on a basis of the captured image data and the three-dimensional shape data.

Preferably, the image capturing section is a CT apparatus, and the captured image data is CT data.

Preferably, the oral cavity scanner is an optical three-dimensional scanner.

Preferably, the processing apparatus creates data about the three-dimensional shape in the oral cavity on a basis of a position of the plate marker included in the captured image data, and a position of the plate marker included in the three-dimensional shape data.

Preferably, the surgical procedure support system includes: a drill frame that has a drill marker, and can be attached to and detached from a drill having a drill blade; a fiducial frame that has a fiducial frame marker, and is attached such that the fiducial frame extends out of the oral cavity from the plate put on the teeth in the oral cavity; an image capturing apparatus that captures images of the drill marker and the fiducial frame marker, and outputs marker image data; a processing apparatus that computes an embedment-completion predicted position of the to-be-embedded object corresponding to a current position of the drill blade, and a current position of the to-be-embedded object corresponding to the current position of the drill blade on a basis of the marker image data and the three-dimensional shape data; and a display apparatus that displays the teeth and a bone where the to-be-embedded object is to be embedded, displays the embedment completion position of the to-be-embedded object decided in advance in a first attribute, displays the embedment-completion predicted position in a second attribute, and displays the current position of the to-be-embedded object in a third attribute.

Preferably, the surgical procedure support system displays a nerve located inside the bone.

Preferably, the surgical procedure support system displays a distance between the nerve and a current tip position of the drill blade.

Preferably, the surgical procedure support system displays a distance between a deepest section of the embedment completion position and a current tip position of the drill blade.

Preferably, the surgical procedure support system displays images of the bone, the embedment completion position, the embedment-completion predicted position and the current position as seen in three different directions.

A surgical procedure support system according to a second invention of the present application includes: a plate that is to be put on teeth, and has a plate marker; an image capturing section that captures an image of a state that the plate is put on the teeth, and outputs captured image data; an oral cavity scanner that acquires a three-dimensional shape in an oral cavity in a state that the plate is put on the teeth, and outputs three-dimensional shape data; a fiducial frame that has a fiducial frame marker, and is attached to the plate such that the fiducial frame extends out of the oral cavity from the plate; a drill frame that has a drill marker, and can be attached to and detached from a drill having a drill blade; a processing apparatus that pre-stores a positional relationship between the plate marker and the fiducial frame marker; an image capturing apparatus that captures images of the drill marker and the fiducial frame marker, and outputs marker image data; and a display apparatus that displays the teeth and a bone where a to-be-embedded object is to be embedded, in which, on a basis of the captured image data, the three-dimensional shape data, the positional relationship between the plate marker and the fiducial frame marker and the marker image data, the processing apparatus computes a positional relationship between the teeth and the fiducial frame marker, and additionally computes an embedment-completion predicted position of the to-be-embedded object corresponding to a current position of the drill blade, and a current position of the to-be-embedded object corresponding to the current position of the drill blade, and the display apparatus displays the teeth and the bone where the to-be-embedded object is to be embedded, displays an embedment completion position of the to-be-embedded object decided in advance in a first attribute, displays the embedment-completion predicted position in a second attribute, and displays the current position of the to-be-embedded object in a third attribute.

A registration apparatus according to a third invention of the present application includes: a plate that is to be put on teeth, and has a plate marker; an image capturing section that captures an image of a state that the plate is put on the teeth, and outputs captured image data; an oral cavity scanner that acquires a three-dimensional shape in an oral cavity in a state that the plate is put on the teeth, and outputs three-dimensional shape data; a fiducial frame that has a fiducial frame marker, and is attached to the plate such that the fiducial frame extends out of the oral cavity from the plate; an image capturing apparatus that captures an image of the fiducial frame marker; and a processing apparatus that pre-stores a positional relationship between the plate marker and the fiducial frame marker, in which the processing apparatus computes a positional relationship between the teeth and the fiducial frame marker on a basis of the captured image data, the three-dimensional shape data and the positional relationship between the plate marker and the fiducial frame marker.

### EFFECT OF THE INVENTION

The present invention provides a surgical procedure support system and a registration apparatus that support a practitioner such that holes where to-be-embedded objects are to be placed can be created accurately.

### BRIEF DESCRIPTION OF THE DRAWINGS

FIG. 1 is a figure schematically depicting a system according to an embodiment of the present invention.
FIG. 2 is a plan view of a plate.
FIG. 3 is a figure depicting the plate and a fiducial frame.
FIG. 4 is a figure depicting the plate and fiducial frame fixed to teeth.
FIG. 5 is a figure depicting a drill and a drill frame.
FIG. 6 is a figure schematically depicting an image displayed on a display apparatus.
FIG. 7 is a flowchart depicting CT processing.
FIG. 8 is a flowchart depicting a first oral cavity scanning process.
FIG. 9 is a flowchart depicting a process of designing positions of implants.
FIG. 10 is a flowchart depicting a process of designing positions of implants.
FIG. 11 is a flowchart depicting a process of performing a procedure with implants.
FIG. 12 is a flowchart depicting a second oral cavity scanning process.
FIG. 13 is a flowchart depicting a third oral cavity scanning process.
FIG. 14 is a flowchart depicting a fourth oral cavity scanning process.
FIG. 15 is a flowchart depicting a frame position computation process.
FIG. 16 is a figure schematically depicting images displayed on the display apparatus.
FIG. 17 is a figure for explaining an abutment tooth formation process.

### DETAILED DESCRIPTION OF THE INVENTION

A surgical procedure support system 100 which is an embodiment of the present invention is explained below with reference to the figures. Explanations are given below on the premise that to-be-embedded objects are implants.

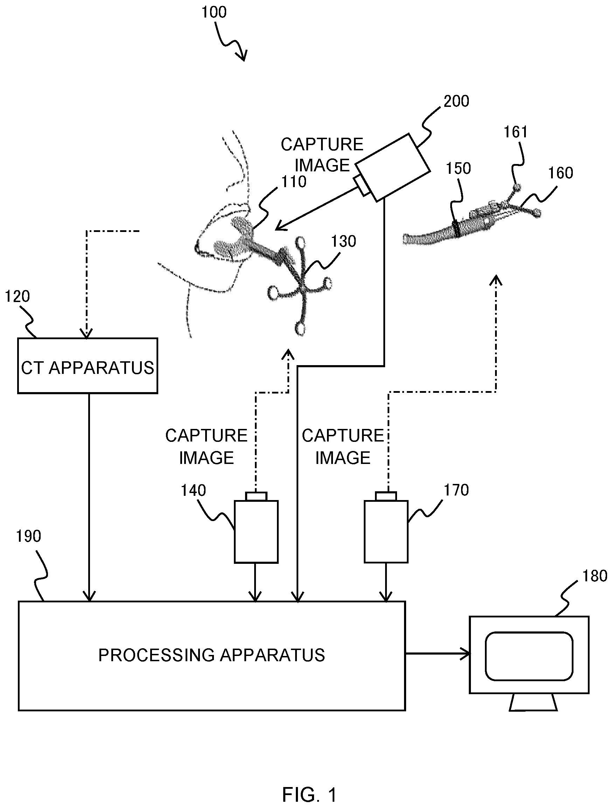

As can be seen by referring to FIG. 1, the surgical procedure support system 100 mainly includes a plate 110, a CT apparatus 120, a fiducial frame 130, a drill 150, a drill frame 160, image capturing apparatuses 140 and 170, a display apparatus 180, a processing apparatus 190 and an oral cavity scanner 200.

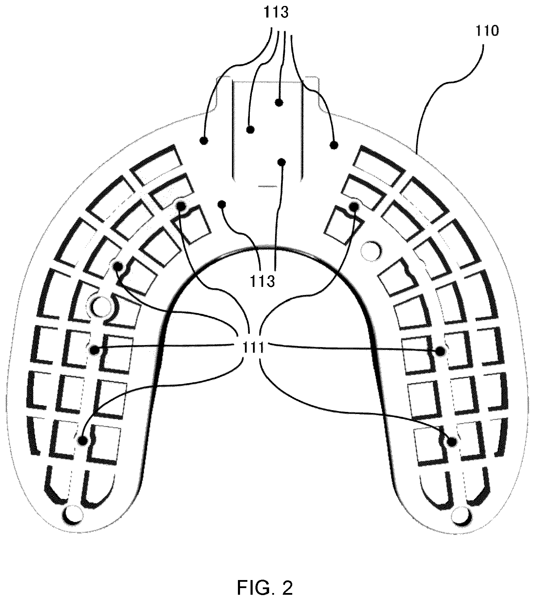

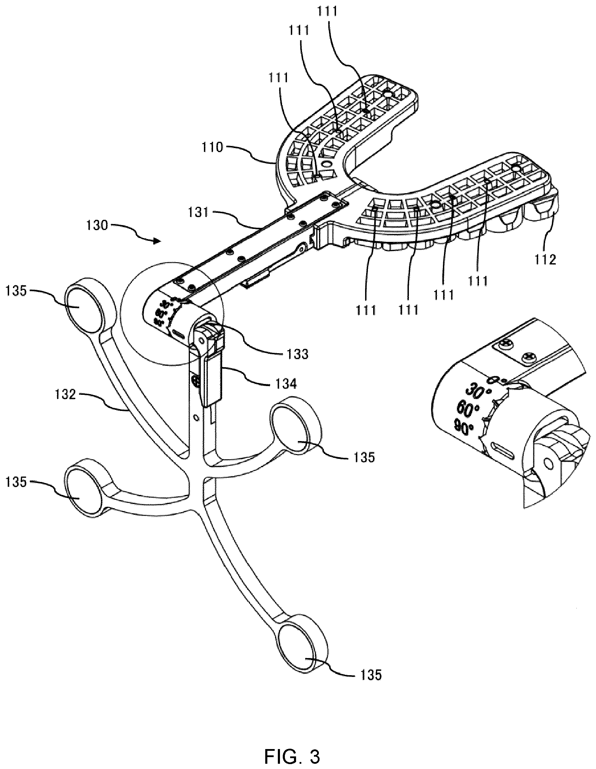

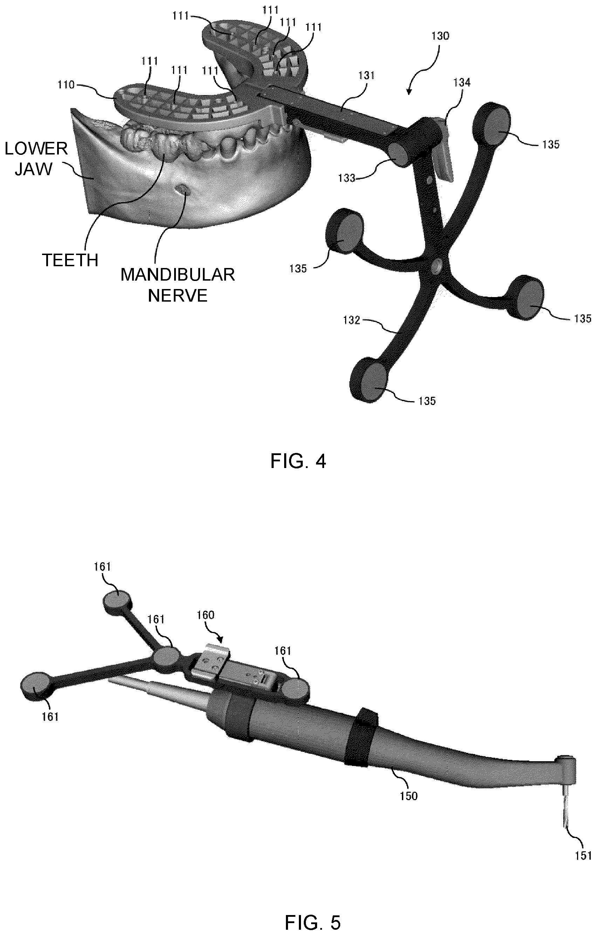

As can be seen by referring to FIGs. 1 to 4, the plate 110 is approximately U-shaped in accordance with the dentition, and is fixed to a stent 112. For example, seven CT markers 111 including titanium balls are embedded inside the plate 110. Optical markers 113 including an optically highly reflective paint is provided on the surface of the plate 110. The positions of the CT markers 111 and optical markers 113 in and on the plate 110 are decided in advance precisely, and are stored on the processing apparatus. The CT markers 111 and optical markers 113 function as plate markers. Then, the plate is put on teeth of a patient via the stent 112 created to match the shapes of the teeth of the patient.

As can be seen by referring to FIG. 1, the CT apparatus 120 is a so-called CT scanning apparatus (image capturing section) that captures a tomographic image of the whole or a part of the area from the upper jaw to the lower jaw of the patient, and outputs CT data (captured image data). Before a hole for an implant is cut, the plate 110 is fixed to the teeth of the patient via the stent 112, and then a tomographic image of the whole or a part of the area from the upper jaw to the lower jaw of the patient is captured along with an image of the plate 110. The CT data obtained by the image-capturing is transmitted to the processing apparatus 190.

The oral cavity scanner 200 is an optical three-dimensional scanner that obtains a stereoscopic image by optically capturing an image of the inside of the oral cavity in a state that the plate 110 is fixed to the teeth by using the stent 112, and outputs the stereoscopic image as three-dimensional shape data. Before the hole for the implant is cut, the plate 110 is fixed to the teeth of the patient via the stent 112, and then the oral cavity scanner 200 captures an image of the whole or a part of the area from the upper jaw to the lower jaw of the patient along with an image of the plate 110. At this time, the plate 110 has a removed portion corresponding to the position where the implant is to be provided. The three-dimensional shape data obtained by the image-capturing by the oral cavity scanner 200 is transmitted to the processing apparatus 190.

As can be seen by referring to FIGs. 1 to 4, the fiducial frame 130 mainly includes a fixation support section 131 that is attached such that the fixation support section 131 extends out of the oral cavity from the plate 110, and a marker support section 132 extending from the fixation support section. The fixation support section 131 and the marker support section 132 are connected via a hinge 133 such that the fixation support section 131 and the marker support section 132 are swingable relative to each other. The swing angle formed between the fixation support section 131 and the marker support section 132 is within the angle range from 0 degrees to 90 degrees, and can be fixed at 0 degrees, 30 degrees, 60 degrees and 90 degrees by positioning a lever 134 attached to the hinge 133 at a fixation position. The fiducial frame 130 is connected with the plate 110 rigidly via an attachment which is not depicted in the figures. Thereby, the positional relationship between the plate 110 and the fiducial frame 130 is represented by a value decided in accordance with the swing angle. Note that the angles at which the swing angle can be fixed by using the lever 134 are an example, and there may be other angles.

The marker support section 132 includes arms extending in four directions from the tip of the marker support section 132, and disk-shaped fiducial frame markers 135 are attached to the arm tips. The fiducial frame markers 135 include a material that reflects infrared rays.

As can be seen by referring to FIG. 1 to FIG. 5, the drill 150 is a dental drill having a tip section to which a drill blade 151 is attached, and the drill frame 160 is attached detachably to a terminal section of the drill 150. The drill frame 160 includes arms extending in three directions from the tip of the drill frame 160, and disk-shaped drill markers 161 are attached to the arm tips. The drill markers 161 include infrared light emitting diodes (IR·LEDs) and a material that reflects infrared rays.

As can be seen by referring to FIG. 1, the image capturing apparatuses 140 and 170 are stereo infrared cameras. The image capturing apparatus 140 captures images of the fiducial frame markers 135, and transmits the obtained images to the processing apparatus 190, and the image capturing apparatus 170 captures images of the drill markers 161, and transmits the obtained images to the processing apparatus 190.

The processing apparatus 190 includes a calculating apparatus and a storage apparatus which are not depicted in the figures, and is connected to the CT apparatus 120, the image capturing apparatuses 140 and 170, the display apparatus 180 and the oral cavity scanner 200. In addition, the processing apparatus 190 has pre-stored thereon a mutual positional relationship of the seven plate markers, and a positional relationship between the plate markers and the fiducial frame markers 135.

On the basis of data from the image capturing apparatuses 140 and 170, the processing apparatus 190 computes an embedment-completion predicted position of an implant corresponding to the current position of the drill blade 151, and the current position of the implant corresponding to the current position of the drill blade 151.

On the basis of CT data and three-dimensional shape data that are obtained by image-capturing performed in a state that the plate 110 is put on the teeth, a positional relationship between the CT markers 111 and the fiducial frame markers 135, and a positional relationship between the drill markers 161 and the fiducial frame markers 135, the processing apparatus 190 computes a positional relationship between the teeth and the fiducial frame markers 135, and additionally computes the embedment-completion predicted position of the implant corresponding to the current position of the drill, and the current position of the implant corresponding to the current position of the drill.

In addition, the processing apparatus 190 stores, at time of designing, an embedment completion position of an implant decided in advance, and the position of a nerve located inside the bone, and, at time of cutting, computes a distance between the nerve and the current tip position of the drill blade 151, and a distance between the deepest section at the embedment completion position and the current tip position of the drill blade 151.

Alternatively, the processing apparatus 190 computes a positional relationship between the teeth and the fiducial frame markers 135 on the basis of CT data and three-dimensional shape data that are obtained by image-capturing performed in a state that the plate 110 is put on the teeth, and the positional relationship between the plate markers and the fiducial frame markers 135. This is called a frame position computation process.

In accordance with signals received from the processing apparatus 190, the display apparatus 180 displays the teeth and the bone where the implant is to be embedded obtained from CT data and three-dimensional shape data, displays the embedment completion position of the implant decided in advance in a first attribute, for example in red, displays the embedment-completion predicted position in a second attribute, for example in yellow, and displays the current position of the implant in a third attribute, for example in blue.

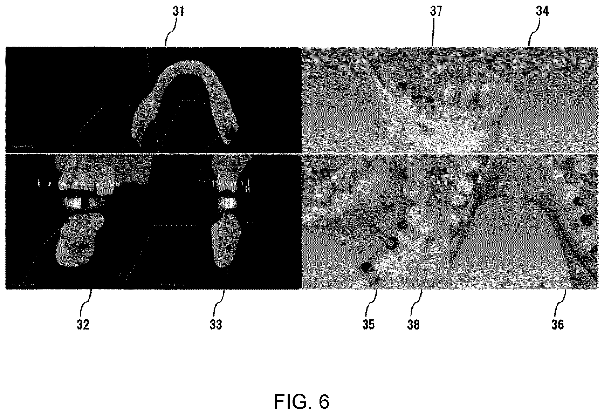

An image displayed on the display apparatus 180 is explained by using FIG. 6. The image is divided into six areas. Three multiplanar reconstruction images (MRP images, multi-planar reconstruction, multi planar reconstruction) obtained from CT data are displayed in left areas 31 to 33, and three 3D rendered images obtained from CT data are displayed in right areas 34 to 36. The display apparatus 180 can display the multiplanar reconstruction images and 3D rendered images as seen in three different directions that are selected from images seen from above, from the outer side, from the inner side, from below, and so on as appropriate in accordance with what a practitioner desires to see. For example, a cross-section specified with the approximately horizontal lines in the crosses depicted in the areas 32 and 33 is displayed in the area 31, a cross-section specified with the approximately vertical lines in the crosses depicted in the areas 31 and 33 is displayed in the area 32, and a cross-section specified with the approximately horizontal line depicted in the area 31, and the approximately vertical line depicted in the area 32 is displayed in the area 33. These approximately horizontal lines and approximately vertical lines are displayed in the same corresponding colors for each cross-section. In addition, a distance 37 between the tip of the drill blade and a bottom section of the embedment completion position, and a distance 38 between the nerve and the tip of the drill blade are displayed in the area 35. Thereby, the practitioner can grasp specific values, and perform precise work.

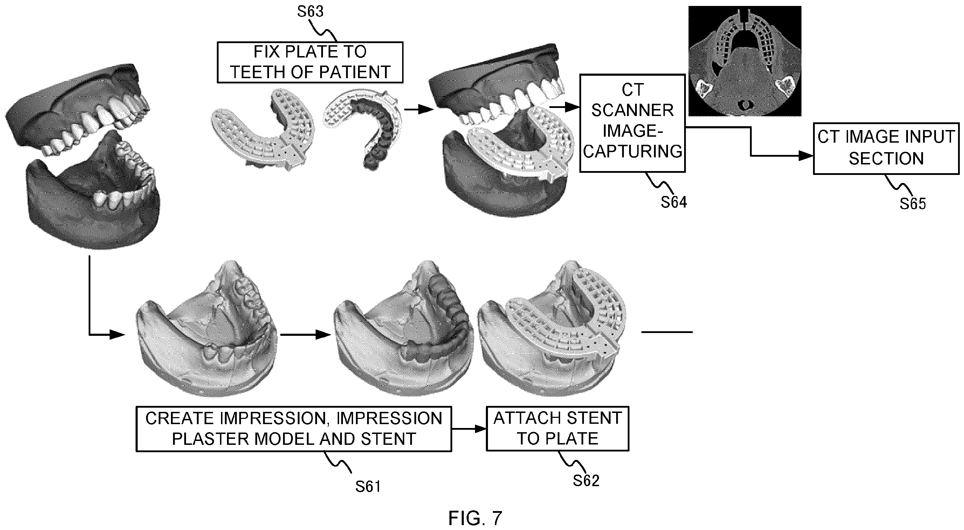

Next, CT processing of capturing a tomographic image of the whole or a part of the area from the upper jaw to the lower jaw of a patient by using the CT apparatus 120, and outputting CT data is explained by using FIG. 7. The CT processing is executed at an installation location of the CT apparatus 120 (e.g. a dental clinic). First, at Step S61, a practitioner takes an impression of teeth where an implant is to be placed, creates a plaster model, and creates the stent 112 by using the plaster model. At next Step S62, the practitioner attaches the stent 112 to the plate 110. Then, at next Step S63, the plate 110 is fixed to the teeth of the patient. At next Step S64, the practitioner captures a tomographic image of the whole or a part of the area from the upper jaw to the lower jaw of the patient along with an image of the plate 110, and obtains CT data. At Step S65, the practitioner transmits the CT data to the processing apparatus 190.

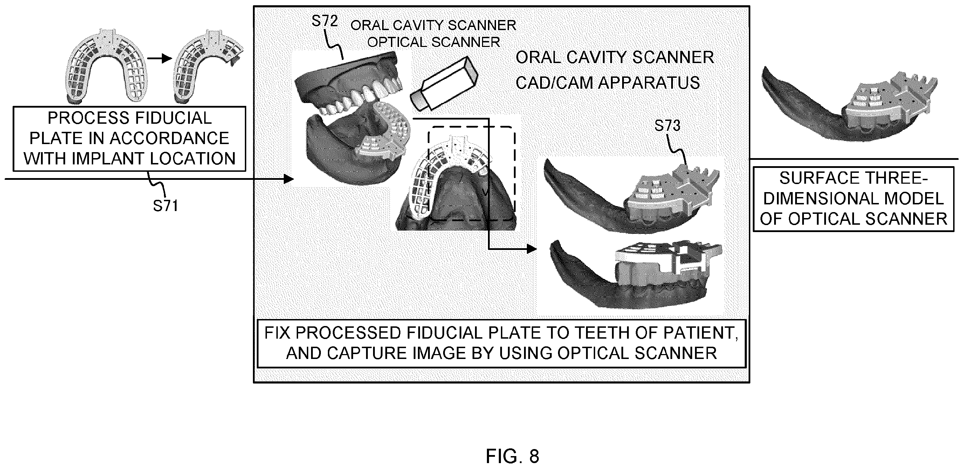

Next, a first oral cavity scanning process of obtaining a stereoscopic image by optically capturing an image of the inside of the oral cavity by using the oral cavity scanner 200, and outputting the image as three-dimensional shape data is explained by using FIG. 8. The first oral cavity scanning process is executed at an installation location of the oral cavity scanner 200 (e.g. a dental clinic). First, at Step S71, the practitioner removes a portion of the plate 110 which corresponds to a position where an implant is to be provided, and fixes the plate 110 to the teeth of the patient. At next Step S72, the practitioner obtains a stereoscopic image by optically capturing an image of the inside of the oral cavity. At next Step S73, the practitioner transmits the stereoscopic image to the processing apparatus 190 as three-dimensional shape data.

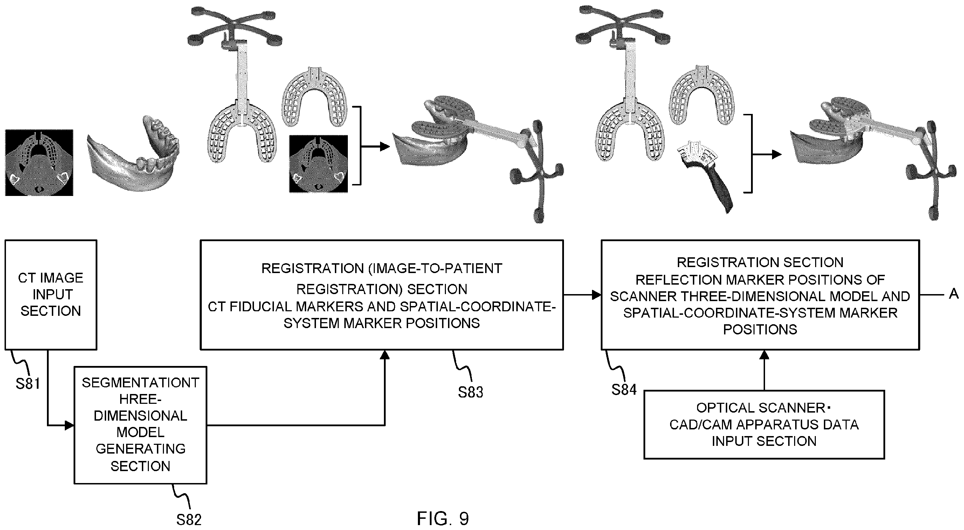

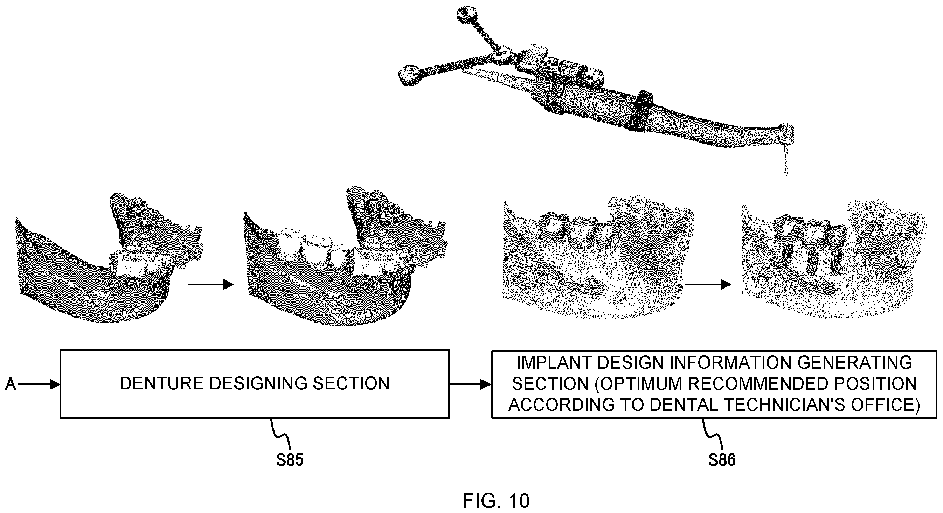

Next, a process of designing a position of the implant by using CT data and three-dimensional shape data is explained by using FIG. 9 and FIG. 10. This process is executed at an installation location of the processing apparatus 190 (e.g. a dental technician's office) by using the processing apparatus 190. First, at Step S81, the processing apparatus 190 obtains CT data from the CT apparatus 120. At next Step S82, the processing apparatus 190 generates a three-dimensional model of the teeth by using the CT data. At next Step S83, the processing apparatus 190 reads out the pre-stored positions of the CT markers 111 of the plate 110, finds the CT markers 111 in the CT data, and records the positions. Then, the processing apparatus 190 registers the pre-stored positions of the CT markers 111 with the positions of the CT markers 111 in the CT data. Thereby, data about a positional relationship between the CT data and the plate 110 is computed. At next Step S84, the processing apparatus 190 obtains three-dimensional shape data from the oral cavity scanner 200, finds the optical markers 113 in the three-dimensional shape data, and records the positions. Then, the processing apparatus 190 registers the pre-stored positions of the optical markers 113 with the positions of the optical markers 113 in the three-dimensional shape data. Thereby, data about a positional relationship between the three-dimensional shape data and the plate 110 is computed. Then, by using the data about the positional relationship between the CT data and the plate 110, and the data about the positional relationship between the three-dimensional shape data and the plate 110, the processing apparatus 190 superimposes an optical three-dimensional image obtained by the oral cavity scanner 200 on a CT image obtained by the CT apparatus 120.

At next Step S85, a user (e.g. a dental technician; a user of the processing apparatus 190 is described as a "dental technician or the like" below) of the processing apparatus 190 refers to the image obtained at Step S84, and designs a position and shape of a denture. At next Step S86, the dental technician or the like designs a position and shape of an implant in accordance with the denture designed at Step S84. Typically, if there is a metallic part in an oral cavity, beam hardening occurs due to the metal, and an accurate CT image cannot be obtained. However, because the processing apparatus 190 superimposes an optical three-dimensional image on a CT image according to the present embodiment, an image of the portion where there is the metallic part and an area surrounding the portion is complemented by the optical three-dimensional image, and an accurate image can be obtained. Then, the dental technician or the like can design a denture and an implant more accurately on the basis of the accurate image.

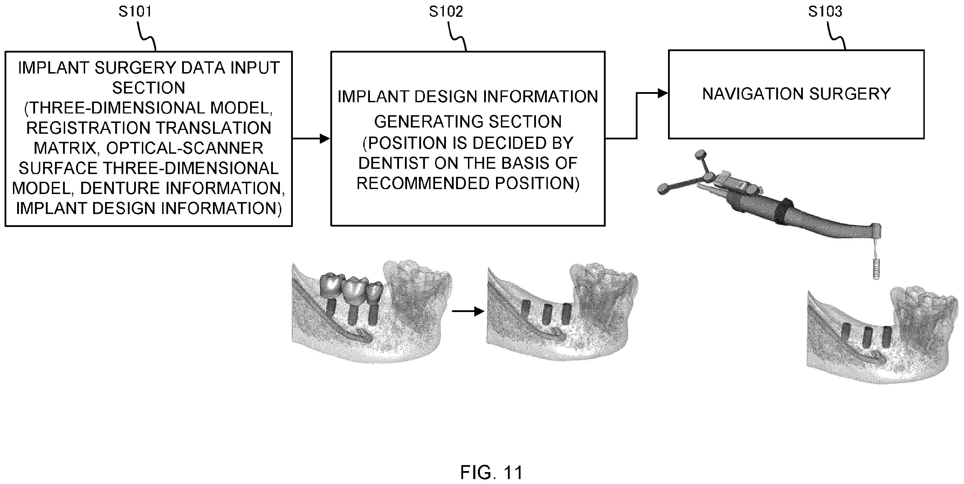

Next, a process of performing a procedure with implants by using the position and shape of the implant designed at Step S86 is explained by using FIG. 11. This process is executed at a dental clinic or the like by a dentist by using a processing apparatus 190 having functionalities similar to those of the processing apparatus 190 used for the process of designing the position of the implant mentioned above. First, at Step S101, the dentist inputs, to the processing apparatus 190, the CT data, the three-dimensional shape data, the position and shape of the denture designed at Step S85, the position and shape of the implant designed at Step S86, and the image obtained at Step S84. At next Step S102, the dentist decides a position of the implant on the basis of the information input at Step S101. Details are mentioned later as a designing process. At next Step S103, the dentist executes an approach process and a cutting process that are mentioned later. Then, the process ends.

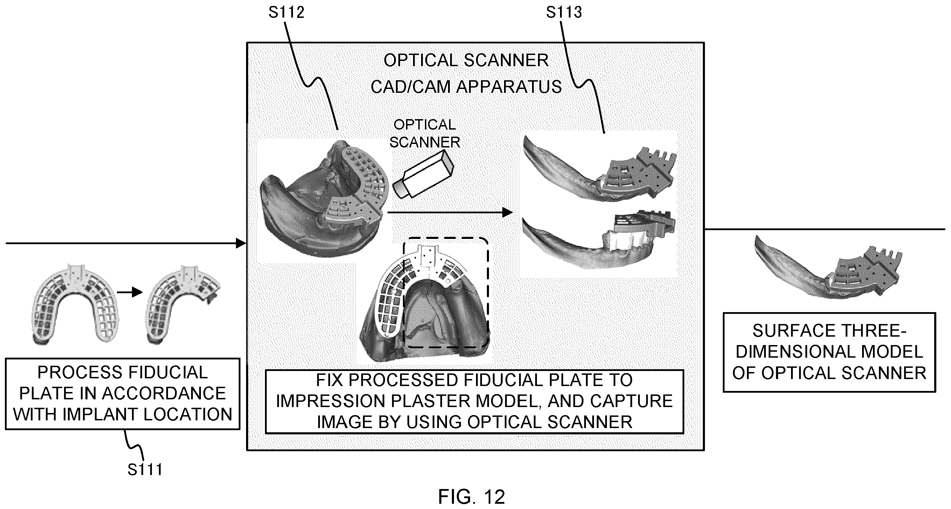

Next, a second oral cavity scanning process which is another embodiment of the first oral cavity scanning process is explained by using FIG. 12. The second oral cavity scanning process is executed at a dental technician's office or the like, similarly to the first oral cavity scanning process. First, at Step S111, a dental technician or the like removes a portion of the plate 110 which corresponds to the position where the implant is to be provided, and fixes the plate 110 to the plaster model created at Step S61. At next Step S112, the dental technician or the like obtains a stereoscopic image by optically capturing an image of the plaster model and the plate 110. At next Step S113, the dental technician or the like transmits the stereoscopic image to the processing apparatus 190 as three-dimensional shape data. Thereby, the dental technician or the like can obtain the stereoscopic image easily and surely.

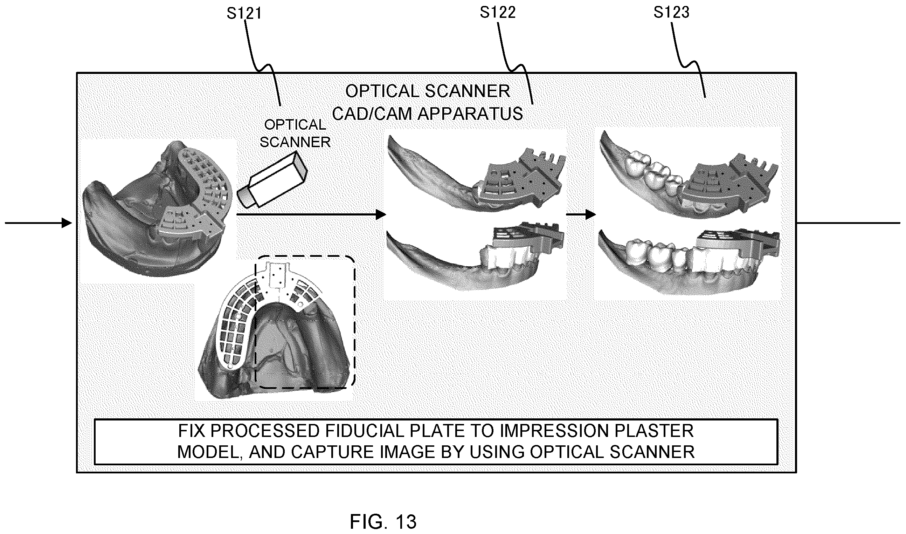

Next, a third oral cavity scanning process which is another embodiment of the first and second oral cavity scanning processes is explained by using FIG. 13. The third oral cavity scanning process is executed at a dental technician's office or the like, similarly to the first oral cavity scanning process. First, at Step S121, a dental technician or the like fixes, to the plaster model created at Step S61, the plate 110 from which a portion corresponding to the position where the implant is to be provided has been removed. At next Step S122, the dental technician or the like obtains three-dimensional shape data by optically capturing an image of the plaster model and the plate 110. At next Step S123, the dental technician or the like creates a denture on the three-dimensional shape data by using CAD/CAM software. Then, the dental technician or the like transmits the created data to the processing apparatus 190 as three-dimensional shape data. Thereby, the dental technician or the like can obtain the three-dimensional shape data including the denture easily and surely.

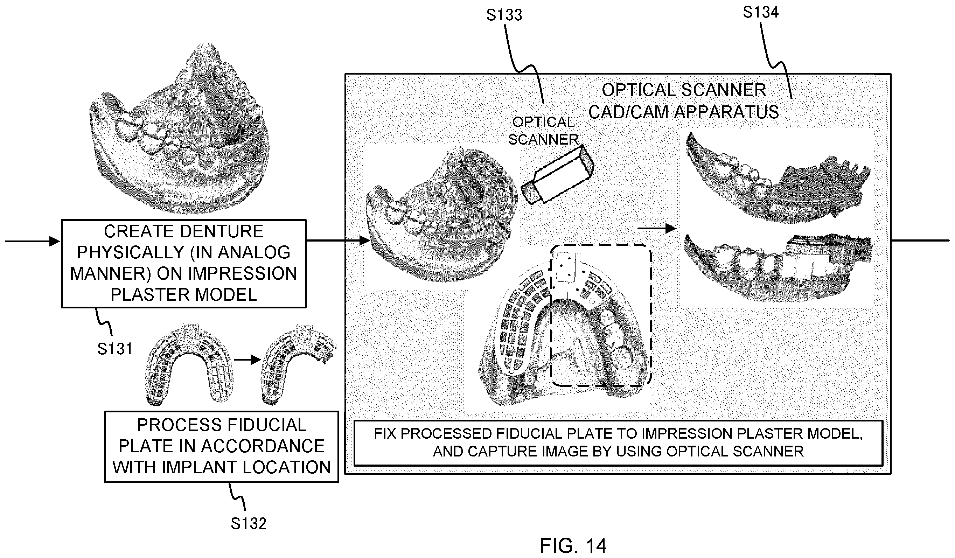

Next, a fourth oral cavity scanning process which is another embodiment of the first to third oral cavity scanning processes is explained by using FIG. 14. The fourth oral cavity scanning process is executed at a dental technician's office or the like, similarly to the first oral cavity scanning process. First, at Step S131, a dental technician or the like creates a denture on the plaster model created at Step S61. At next Step S132, a dental technician or the like removes a portion of the plate 110 which corresponds to the position where the implant is to be provided, and fixes the plate 110 to the plaster model including the denture created at Step S131. At next Step S133, the dental technician or the like obtains a stereoscopic image by optically capturing an image of the plaster model and the plate 110. At next Step S134, the dental technician or the like transmits the stereoscopic image to the processing apparatus 190 as three-dimensional shape data. Thereby, the dental technician or the like can obtain the stereoscopic image including the denture easily and surely.

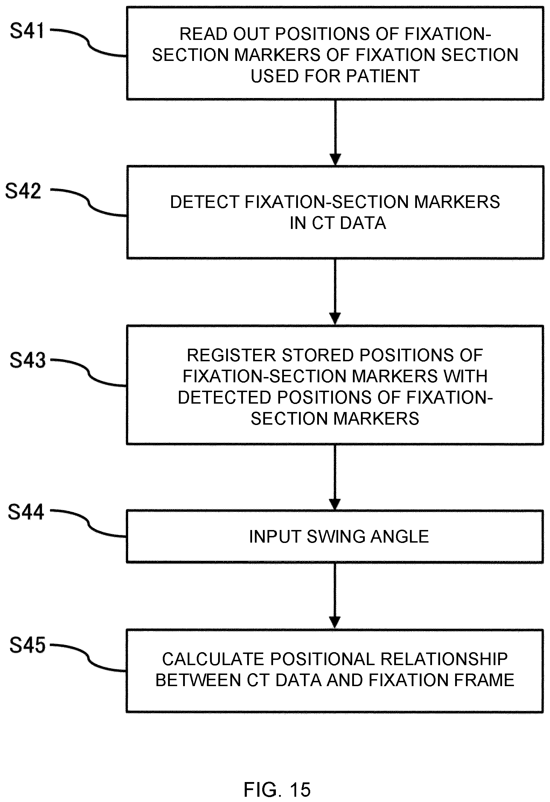

Next, the frame position computation process is explained by using FIG. 15. At Step S41, the processing apparatus 190 reads out the pre-stored positions of the CT markers 111 in the plate 110. Next, at Step S42, the processing apparatus 190 finds the CT markers 111 in the CT data obtained by image-capturing performed in a state that the plate 110 is put on the teeth, and records the positions. At next Step S43, the processing apparatus 190 registers the positions of the CT markers 111 read out at Step S41 with the positions of the CT markers 111 found at Step S42. Thereby, data about a positional relationship between the CT data and the plate 110 is computed. At next Step S44, the practitioner inputs, to the processing apparatus, a swing angle formed between the fixation support section 131 and the marker support section 132 which is any one of 0 degrees, 30 degrees, 60 degrees and 90 degrees. At next Step S45, the processing apparatus 190 first determines a determinant determined in accordance with the swing angle input at Step S44. Here, because the positional relationship between the plate 110 and the fiducial frame 130 is represented by a known value as mentioned before, the processing apparatus 190 computes the determinant determined in accordance with the swing angle on the basis of the positional relationship between the plate markers and the fiducial frame markers 135. Then, the processing apparatus 190 multiplies the data about the positional relationship between the CT data and the plate 110 decided at Step S43 by the determinant, and calculates a positional relationship between the CT data and the fiducial frame 130. Then, the process ends.

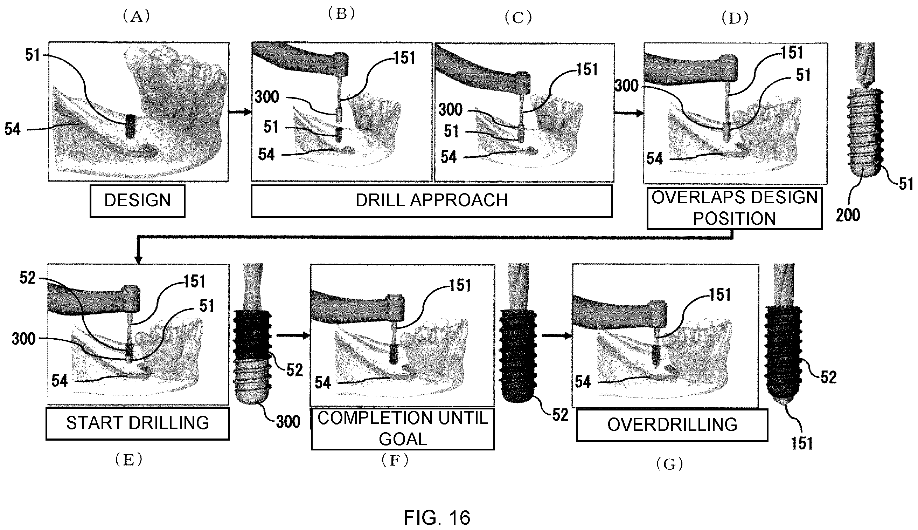

Next, an implant procedure process using the surgical procedure support system 100 is explained by using FIG. 16. The procedure process includes the designing process, the approach process and the cutting process. First, these are explained in this order below.

The designing process is a process of deciding a position of the implant on the basis of CT data. First, the practitioner determines the position of a nerve 54 located inside the bone on the basis of the CT data. Then, the practitioner decides the position of the implant taking care that the position of the implant does not overlap the position of the nerve 54, and additionally taking various factors into consideration. Then, the practitioner inputs, to the processing apparatus 190, data about the positions of the nerve 54 and implant.

The approach process is a process of deciding an entrance angle of the drill blade 151 relative to the bone. First, the processing apparatus 190 calculates the teeth and the bone where the implant is to be embedded, an embedment completion position 51 of the implant decided in advance, and the position of the nerve 54, and creates a synthesized image of these. Next, the display apparatus 180 displays the image (see (A) in FIG. 16). At this time, the embedment completion position 51 is displayed in red. Next, referring to the image displayed on the display apparatus 180, the practitioner causes the drill blade 151 to enter the inside of the oral cavity of the patient, and tentatively presses the drill blade 151 against a position where the implant is planned to be provided. At this time, the image capturing apparatus 140 captures images of the fiducial frame markers 135, and consecutively transmits the obtained images to the processing apparatus 190, and the image capturing apparatus 170 captures images of the drill markers 161, and consecutively transmits the obtained images to the processing apparatus 190. The processing apparatus 190 calculates the position of the drill blade 151 in the oral cavity of the patient by using the images, and, on the basis of the determined position, causes the display apparatus 180 to display the position of the drill blade 151 relative to the bone consecutively along with a virtual implant 300 added as a virtual object to the tip of the drill blade 151 (see (B) FIG. 16). The virtual implant 300 represents an embedment-completion predicted position representing a prediction of the position where the implant is to be embedded, and is displayed in yellow. Then, when the practitioner operates the drill, and changes the position and angle of the drill blade 151 relative to the bone, the processing apparatus 190 changes the positions and angles of the drill blade 151 and virtual implant 300 displayed on the display apparatus 180 in accordance with the changes (see (C) and (D) in FIG. 16). When the practitioner makes sure that the position and angle of the virtual implant 300 match the embedment completion position 51, the procedure proceeds to the next cutting process.

The cutting process is a process of actually creating a hole in the bone of the patient. Subsequent to the approach process, while the state that the position and angle of the virtual implant 300 are matching the embedment completion position 51 is maintained, the practitioner operates the drill blade 151, and starts cutting (see (E) in FIG. 16). In this process also, similarly to the approach process, the image capturing apparatuses 140 and 170, the processing apparatus 190 and the display apparatus 180 operate consecutively, and the processing apparatus 190 causes the display apparatus 180 to consecutively display, as a current position 52 and in blue, the position of the drill blade 151 entering the inside of the bone. Because each position is displayed on the display apparatus 180 in the consecutively updated state, if the current position 52 does not match the position and angle decided at the approach process, the virtual implant 300 shifts away from the embedment completion position 51 on the display apparatus 180. Therefore, the practitioner who saw this can easily sense that if the practitioner continues the cutting without changing the current angle, a hole is created undesirably at a position off the embedment completion position 51, and furthermore can correct the current angle to the appropriate entrance angle. Then, the practitioner performs the cutting until the current position 52 matches the embedment completion position 51 (see (F) in FIG. 16), and completes the cutting process. Note that in a case that the current position 52 goes beyond the embedment completion position 51, the drill blade 151 sticks out from a bottom section of the embedment completion position 51 (see (G) FIG. 16). Thereby, the practitioner can easily sense that the current position 52 has gone beyond the embedment completion position 51, and can stop the cutting. According to the process above, the practitioner can grasp the position and angle of the drill blade 151 while seeing the image displayed on the display apparatus 180, and perform the cutting while correcting the position and angle as appropriate.

Note that the number of plate markers is not limited to seven, but only has to be a number greater than one. In addition, the drill markers 161 may include either infrared light emitting diodes (IR·LEDs) or a material that reflects infrared rays.

The swing angle is not limited to an angle within the angle range from 0 degrees to 90 degrees or 0 degrees, 30 degrees, 60 degrees or 90 degrees, but may be fixed at an angle within another angle range or at angles other than them.

Note that whereas it is supposed in the explanation that there is one processing apparatus 190, one processing apparatus 190 may be installed at a dental technician's office, and another separate processing apparatus 190 may be installed at a dental clinic as a surgical procedure support apparatus.

An explanation has mainly been given thus far on the premise that an implant is embedded and placed in the oral cavity of a patient. As mentioned above, objects to be placed in the oral cavity of a patient are not limited to implants, but may be teeth of the patient her/himself. That is, the present invention can be applied also to tooth transplant navigation. In this case, in FIG. 16, instead of the virtual implant 300, the shape of a to-be-transplanted tooth of the patient is displayed on the display apparatus 180. In addition, instead of the embedment completion position 51 of the implant, the shape of the to-be-transplanted tooth of the patient is displayed as a copy on the display apparatus 180. Each tooth has a different shape, and also has a more complicated shape than the shapes of implants. After attaining an immediately-extractable state (dislocated state) of the to-be-transplanted tooth at its pre-transplant position, the practitioner cuts in advance the bone at a position where the tooth is to be transplanted, in accordance with the display on the display apparatus 180. Thereby, the to-be-transplanted tooth at its pre-transplant position can be transplanted promptly, and so the success rate of the tooth transplant can be enhanced.

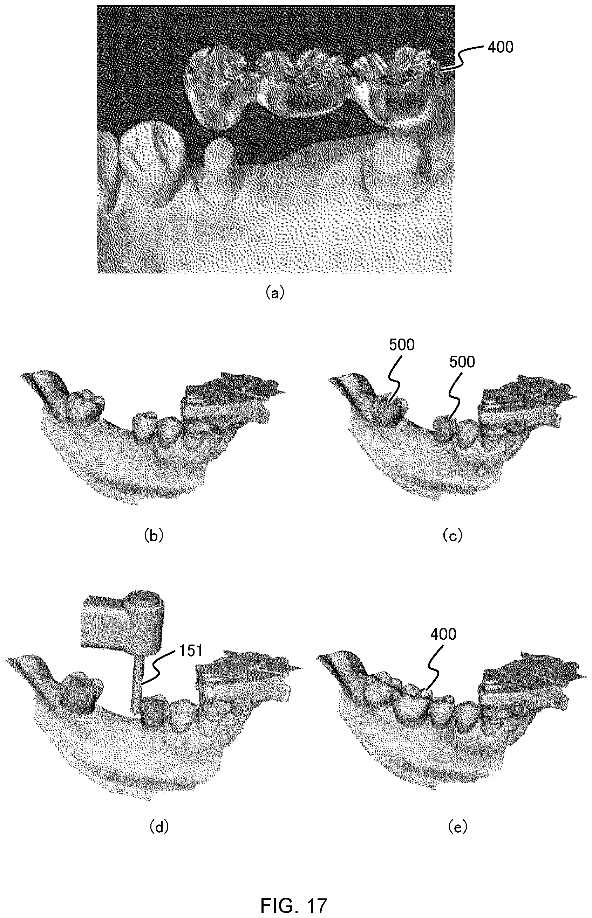

A system that supports embedment of a to-be-embedded object by using both three-dimensional shape data obtained at the CT apparatus 120, and three-dimensional shape data obtained at the oral cavity scanner 200 has been explained thus far. In a case that a to-be-embedded object such as an implant is embedded in the oral cavity of a patient, a part of the bone of the patient needs to be cut, and so three-dimensional shape data obtained at the CT apparatus 120 and including the bone of the patient is necessary. In contrast to this, in abutment tooth formation performed for placing a single full crown or bridge on teeth of a patient, data including a captured image of an area up to the bone of the practitioner is not necessary. Because of this, only three-dimensional shape data obtained at the oral cavity scanner 200 may be used in the abutment tooth formation. This case is explained below.
(a) to (e) in FIG. 17 are figures for explaining the abutment tooth formation process. Specifically, (a) in FIG. 17 is a figure depicting a bridge 400 and abutment teeth 500. As depicted in (a) in FIG. 17, in the abutment tooth formation process, a practitioner cuts parts of teeth of a patient in order to fit the bridge 400 to the teeth of the patient.
(b) in FIG. 17 is a figure depicting three-dimensional shape data of the oral cavity surface of the patient reconstructed from data of the oral cavity scanner 200, and depicts a video displayed on the display apparatus 180. The practitioner first captures an image of the inside of the oral cavity of the patient by using the oral cavity scanner 200, and generates three-dimensional shape data.
(c) in FIG. 17 is a figure depicting a design for the abutment tooth formation, and is equivalent to the designing process in FIG. 16. As depicted in (c) in FIG. 17, the practitioner decides shapes of the abutment teeth 500 taking the properties of the bridge 400 and crown used for the procedure into consideration. Then, the practitioner inputs the decided shapes of the abutment teeth 500 to the processing apparatus 190. The display apparatus 180 displays the shapes of the abutment teeth 500 decided by the practitioner in a first attribute (e.g. in red) which is different from other attributes.
(d) in FIG. 17 is a figure equivalent to the cutting process in FIG. 16. The image capturing apparatuses 140 and 170, the processing apparatus 190 and the display apparatus 180 operate consecutively, and the processing apparatus 190 causes the display apparatus 180 to consecutively display, as the current position 52 and in a second attribute (e.g. in blue), the position of the drill blade 151 approaching teeth that should be formed into the abutment teeth 500. Because the shapes of the abutment teeth 500 decided by the practitioner are displayed in the first attribute, the practitioner can operate the drill blade 151 while seeing the display on the display apparatus 180, and cut the teeth until the teeth have the decided shapes of the abutment teeth 500.
(e) in FIG. 17 is a figure depicting a state that the bridge 400 is attached to the abutment teeth 500. By using a plaster model of the teeth of a practitioner instead of the actual teeth of a patient, the surgical procedure support system 100 according to the embodiment can also be used for training of the abutment tooth formation by practitioners.

Whereas the present invention has been explained by using embodiments thus far, the technical scope of the present invention is not limited by the scope described in the embodiments described above, but various modifications and changes are possible within the scope of a gist of the present invention. For example, all or some of apparatuses can be configured functionally or physically distributed or integrated in any units. In addition, new embodiments that are generated by any combination of a plurality of embodiments are also included in embodiments of the present invention. Effects of the new embodiments generated by the combination include the effects of the original embodiments.

Note that the size, shape and quantity of each member depicted in the present specification and figures are depicted as examples, and these size, shape and quantity are not the sole examples. In addition, the material of each member is depicted as an example, and the material is not the sole example.

Whereas embodiments of the present invention have been explained with reference to the figures attached here, it is apparent for those skilled in the art that modifications are made in the structure and relationship of each section without deviating from the described scope and spirit of the invention.

### DESCRIPTION OF THE REFERENCE NUMERALS

100: Surgical procedure support system
110: Plate
111: CT marker
112: Stent
113: Optical marker
120: CT apparatus
130: Fiducial frame
131: Fixation support section
132: Marker support section
133: Hinge
134: Lever
135: Fiducial frame marker
140: Image capturing apparatus
150: Drill
151: Drill blade
160: Drill frame
161: Drill marker
170: Image capturing apparatus
180: Display apparatus
190: Processing apparatus
200: Oral cavity scanner
300: Virtual implant

## Claims

1. A surgical procedure support system comprising:
a plate that is to be put on teeth, and has a plate marker;
an image capturing section that captures an image of a state that the plate is put on the teeth, and outputs captured image data;
an oral cavity scanner that acquires a three-dimensional shape in an oral cavity in a state that the plate is put on the teeth, and outputs three-dimensional shape data; and
a processing apparatus that computes an embedment completion position of a to-be-embedded object on a basis of the captured image data and the three-dimensional shape data.

2. The surgical procedure support system according to claim 1, wherein the image capturing section is a CT apparatus, and the captured image data is CT data.

3. The surgical procedure support system according to claim 1 or 2, wherein the oral cavity scanner is an optical three-dimensional scanner.

4. The surgical procedure support system according to any of claims 1 to 3, wherein the processing apparatus creates data about the three-dimensional shape in the oral cavity on a basis of a position of the plate marker included in the captured image data, and a position of the plate marker included in the three-dimensional shape data.

5. The surgical procedure support system according to claim 4, comprising:
a drill frame that has a drill marker, and can be attached to and detached from a drill having a drill blade;
a fiducial frame that has a fiducial frame marker, and is attached such that the fiducial frame extends out of the oral cavity from the plate put on the teeth in the oral cavity;
an image capturing apparatus that captures images of the drill marker and the fiducial frame marker, and outputs marker image data;
a processing apparatus that computes an embedment-completion predicted position of the to-be-embedded object corresponding to a current position of the drill blade, and a current position of the to-be-embedded object corresponding to the current position of the drill blade on a basis of the marker image data and the three-dimensional shape data; and
a display apparatus that displays the teeth and a bone where the to-be-embedded object is to be embedded, displays the embedment completion position of the to-be-embedded object decided in advance in a first attribute, displays the embedment-completion predicted position in a second attribute, and displays the current position of the to-be-embedded object in a third attribute.

6. The surgical procedure support system according to claim 5, wherein a nerve located inside the bone is displayed.

7. The surgical procedure support system according to claim 6, wherein a distance between the nerve and a current tip position of the drill blade is displayed.

8. The surgical procedure support system according to claim 6 or 7, wherein a distance between a deepest section of the embedment completion position and a current tip position of the drill blade is displayed.

9. The surgical procedure support system according to any of claims 5 to 8, wherein images of the bone, the embedment completion position, the embedment-completion predicted position and the current position as seen in three different directions are displayed.

10. A surgical procedure support system comprising:
a plate that is to be put on teeth, and has a plate marker;
an image capturing section that captures an image of a state that the plate is put on the teeth, and outputs captured image data;
an oral cavity scanner that acquires a three-dimensional shape in an oral cavity in a state that the plate is put on the teeth, and outputs three-dimensional shape data;
a fiducial frame that has a fiducial frame marker, and is attached to the plate such that the fiducial frame extends out of the oral cavity from the plate;
a drill frame that has a drill marker, and can be attached to and detached from a drill having a drill blade;
a processing apparatus that pre-stores a positional relationship between the plate marker and the fiducial frame marker;
an image capturing apparatus that captures images of the drill marker and the fiducial frame marker, and outputs marker image data; and
a display apparatus that displays the teeth and a bone where a to-be-embedded object is to be embedded, wherein
on a basis of the captured image data, the three-dimensional shape data, the positional relationship between the plate marker and the fiducial frame marker and the marker image data, the processing apparatus computes a positional relationship between the teeth and the fiducial frame marker, and additionally computes an embedment-completion predicted position of the to-be-embedded object corresponding to a current position of the drill blade, and a current position of the to-be-embedded object corresponding to the current position of the drill blade, and
the display apparatus displays the teeth and the bone where the to-be-embedded object is to be embedded, displays an embedment completion position of the to-be-embedded object decided in advance in a first attribute, displays the embedment-completion predicted position in a second attribute, and displays the current position of the to-be-embedded object in a third attribute.

11. A registration apparatus comprising:
a plate that is to be put on teeth, and has a plate marker;
an image capturing section that captures an image of a state that the plate is put on the teeth, and outputs captured image data;
an oral cavity scanner that acquires a three-dimensional shape in an oral cavity in a state that the plate is put on the teeth, and outputs three-dimensional shape data;
a fiducial frame that has a fiducial frame marker, and is attached to the plate such that the fiducial frame extends out of the oral cavity from the plate;
an image capturing apparatus that captures an image of the fiducial frame marker; and
a processing apparatus that pre-stores a positional relationship between the plate marker and the fiducial frame marker, wherein
the processing apparatus computes a positional relationship between the teeth and the fiducial frame marker on a basis of the captured image data, the three-dimensional shape data and the positional relationship between the plate marker and the fiducial frame marker.
